**Europäisches Patentamt**

**European Patent Office**

**Office européen des brevets**

(19)

(11) Veröffentlichungsnummer: **0 119 420**
**B1**

(12) # EUROPÄISCHE PATENTSCHRIFT

(45) Veröffentlichungstag der Patentschrift:
14.01.87

(51) Int. Cl.⁴: **C 08 B 37/16**

(21) Anmeldenummer: **84101061.4**

(22) Anmeldetag: **02.02.84**

(54) **In Wasser schnell und stark quellende Cyclodextrinpolymere, Verfahren zu deren Herstellung und deren Verwendung als Desintegrationsmittel für die Tablettenherstellung.**

(30) Priorität: **04.02.83 HU 38783**

(43) Veröffentlichungstag der Anmeldung:
**26.09.84 Patentblatt 84/39**

(45) Bekanntmachung des Hinweises auf die Patenterteilung:
**14.01.87 Patentblatt 87/3**

(84) Benannte Vertragsstaaten:
**AT CH DE FR GB LI**

(56) Entgegenhaltungen:
JOURNAL OF THE AMERICAN CHEMICAL
SOCIETY, Band 104, Nr. 23, 1982, Seiten 6283-6288,
R.I. GELB et al.: "Cyclohexaamylose complexation
with organic solvent molecules"
DIE STÄRKE; Band 21, Nr. 5, Mai 1969, Seiten 119-123
N. WIEDENHOF et al.: "Properties of cyclodextrins.
Part III. Cyclodextrin-epichlorhydrin resins:
Preparation and analysis"
CHEMICAL ABSTRACTS, Band 97, Nr. 22,
November 1982, Seite 381, Nr. 188198a, Columbus,
Ohio, USA E. KOROS et al.: "Preliminary studies on
tablet disintegration by sorption calorimetry"

(73) Patentinhaber: **CHINOIN Gyogyszer és Vegyészeti
Termékek Gyára RT., To utca 1-5, H-1045
Budapest IV (HU)**

(72) Erfinder: **Fenyvesi, Eva, Dr., Szántó B.u. 5/b,
H-1145 Budapest (HU)**
Erfinder: **Szejtli, József, Dr., Endrödi S.u. 38-40,
H-1026 Budapest (HU)**
Erfinder: **Zsadon, Béla, Dr., Villányi u. 64/b, H-1113
Budapest (HU)**
Erfinder: **Antal, Balázs, Damjanich u. 42, H-1071
Budapest (HU)**
Erfinder: **Wágner geb. Koháry, Ildikó, Sallai Imre
u. 6, H-1136 Budapest (HU)**

(74) Vertreter: **Lotterhos, Hans Walter, Dr.- Ing.,
Lichtensteinstrasse 3, D-6000 Frankfurt am Main
1 (DE)**

**0 119 420**

## Beschreibung

Die Erfindung betrifft Cyclodextrin-polymere, die in Wasser schnell und in starkem Maße quellen, sowie ein Verfahren zu deren Herstellung.

Die Wasserunlöslichen Cyclodextrin-polymere werden durch Vernetzungspolymerisation der Cyclodextrine hergestellt. Als Vernetzungsmittel werden meistens Epichlorhydrin oder Diepoxyverbindungen (z.B. Diepoxybutan, Diepoxypropyläther, Äthylenglykol-diepoxypropyläther) verwendet, und zwar unabhängig davon, ob das Polymer durch Blockpolymerisation (NL-A- 6 505 361, US-A- 3 472 835), in Form von regulären Perlen (GB-PS 1 244 990) oder in Form von größeren, spezifische Oberfläche aufweisenden, geschäumten Produkten (CH-A- 653 351) hergestellt wird. Die mechanischen Eigenschaften der sonst spröden Substanzen werden durch Einbau von Polyvinylalkohol wesentlich verbessert (US-A- 4 274 985), was insbesondere bei der Anwendung als stationäre Phase in der Chromatographie wichtig sind.

Die Wasseraufnahme der nach bekannten Verfahren hergestellten Cyclodextrin-polymere kann innerhalb weiter Grenzen variiert werden, und zwar durch Variation des Überschusses an Vernetzungsmittel und der Ausgangskonzentration an Cyclodextrin. Die Wasseraufnahme des Produktes ist größer, wenn ein größerer Überschuß an Vernetzungsmittel angewandt oder wenn die Vernetzung in stärker verdünnten Lösungen durchgeführt wird. Die so hergestellten Polymere mit größerem Wasseraufnahmevermögen nehmen aber das Wasser nur langsam auf, und die vollständige Quellung erfordert in einigen Fällen sogar mehrere Stunden. Das Wasseraufnahmevermögen der in an sich bekannter Weise hergestellten, schnell quellenden Cyclodextrin-polymere ist gering, und bei Polymeren mit großem Wasseraufnahmevermögen ist die Geschwindigkeit der Wasseraufnahme niedrig. Infolgedessen lassen sich nach den bekannten Verfahren keine Polymere herstellen, die schnell eine große Menge Wasser aufnehmen.

Das Ziel der Erfindung ist die Entwicklung eines Verfahrens zur Herstellung schnell quellender Cyclodextrin-Polymere mit großem Wasseraufnahmevermögen.

Es wurde überraschender Weise gefunden, daß sich Cyclodextrin-polymere, die diesen Erfordernissen entsprechen in einfacher Weise durch Umsetzung von Cyclodextrin und Epichlorhydrin in wäßrig-alkalischem Medium herstellen lassen, wenn man auf 1 Mol Cyclodextrin 10 bis 20 Mol Alkalimetallhydroxyd, 10 bis 20 Mol Epichlorhydrin, 2 bis 8 Mol einer Alkohol-Zusatzkomponente, vorzugsweise ein $C_2$-$C_8$-Alkohol, ein Glykol-derivat oder Glycerin, und gegebenenfalls 10 bis 15 Mol eines organischen Lösungsmittels, das bei der Reaktionstemperatur verdampft, vorzugsweise Dichloräthan, oder 8 bis 12 Mol eines hochsiedenden Lösungsmittels, das eine heterogene Phase sichert anwendet.

In den Verfahren der Erfindung können als Cyclodextrin α -, β- oder α-Cyclodextrin oder deren Gemisch verwendet werden. Die Art des Cyclodextrins beeinflußt das Quellvermögen des erhaltenen Produkts nicht wesentlich.

Das Verfahren der Erfindung wird in wäßrig-alkalischem Medium durchgeführt, wobei die alkalische Reaktion des Reaktionsgemisches durch Alkalimetallhydroxyde gewährleistet wird.

Die Bildung der gewünschten Polymere mit den vorteilhaften Eigenschaften kann gemäß Erfindung dadurch gesichert werden, daß der im Reaktionsgemisch vorhandene Alkohol-Zusatz ($C_2$-$C_8$-Alkohol, Glykol-Derivat oder Glycerin) mit Hilfe des Epichlorhydrins in die Polymerstruktur eingebaut wird, was zu einer Auflockerung dieser Struktur führt, wodurch sowohl die Wasseraufnahme als auch das Quellvermögen des Polymers günstig beeinflußt wird. Diese Wirkung ist unerwartet und überraschend, weil die Polymere der HU-B- 177 419, die Polyvinylalkohol eingebaut enthalten, nur eine verbesserte mechanische Festigkeit nicht aber ein größeres Wasseraufnahme- oder Quellvermögen als die Polymere ohne Polyvinylalkohol aufweisen.

Im Verfahren der Erfindung können als Alkohol-Zusatz $C_2$-$C_8$-Alkohole und Glykole, wie Äthanol, Propanol, Butanol, Pentanol, Hexanol, Heptanol, Octanol, Äthylenglykol, Propylenglykol, Butylenglykol und andere Glykole sowohl in Form der normalen wie auch der verzweigten Isomeren sowie auch Triäthylenglykol, Tetraäthylenglykol und Glycerin verwendet werden.

Die Polymerisationsreaktion gemäß Erfindung wird zweckmäßig bei einer Temperatur von 60 bis 80°C durchgeführt; es sind aber auch höhere oder niedrigere Temperaturen möglich.

Sollen Produkte mit Schaumstruktur hergestellt werden, wird dem Reaktionsgemisch außerdem ein schaumerzeugender Zusatz zugegeben, z.B. bei der Reaktionstemperatur siedende Lösungsmittel (wie Dichloräthan). Sollen Perlpolymerisate hergestellt werden, wird dem Reaktionsgemisch ein mit Wasser nicht mischbares, hochsiedendes Lösungsmittel, vorzugsweise Toluol, zugesetzt.

Die Polymere der Erfindung quellen in Wasser schnell und in starkem Maße, so daß sie mit Erfolg als Desintegratoren bei der Herstellung von Tabletten (s. Tabelle IV) verwendet werden können.

Weitere Einzelheiten der Erfindung sind den folgenden Beispielen zu entnehmen. Hieraus sind keine Beschränkungen herzuleiten.

## Beispiel 1

3,0 g (75 mMol) Natriumhydroxyd und danach 5,0 g (4,4 mMol) β-Cyclodextrin löst man in 10 ml Wasser von 60°C und mischt dem erhaltenen Reaktionsgemisch zunächst 2,5 ml (2,8 g, 14,5 mMol) Tetraäthylenglykol und danach 5,7 ml (6,7 g, 72,7 mMol) Epichlorhydrin bei 60°C innerhalb von etwa 1,5 Stunden zu. Nach dem

2

Abkühlen wird das erhaltene Gel mit destilliertem Wasser salzfrei gewaschen, durch Behandlung mit Aceton dehydratisiert, bei 105°C getrocknet und pulverisiert. Man erhält 6,8 g eines weißen, pulverigen Produkts (Produkt A), was einen Cyclodextringehalt von 50,8 % (jodometrisch bestimmt) aufweist.

Herstellung von Cyclodextrin-polymer mit dem gleichen Wasseraufnahmevermögen nach bekannten Verfahren, ohne Alkohol-Zusatz:

In 10 ml Wasser von 60°C werden zunächst 2,2 g (55 mMol) Natriumhydroxyd, sodann 5,0 g (4,4 mMol) β-Cyclodextrin gelöst und dann dem erhaltenen Reaktionsgemisch 4,2 ml (5,0 g, 54 mMol) Epichlorhydrin innerhalb von etwa 1,5 Stunden zugegeben. So werden 5,3 g eines weißen, pulverigen Produkts (Produkt B) erhalten, dessen Cyclodextringehalt 53,4 % beträgt.

**Beispiel 2**

In 30 ml Wasser von 60°C löst man 4,4 g (110 mMol) Natriumhydroxyd, danach 25 g (22 mMol) β-Cyclodextrin und mischt der erhaltenen Lösung zunächst 9,0 ml (10,0 g, 161 mMol) Äthylenglykol und dann 8,5 ml (10,0 g, 108 mMol) Epichlorhydrin zu. Das Reaktionsgemisch hält man eine Stunde bei 60°C und mischt danach 20 ml Dichloräthan als schaumerzeugendes Mittel zu. Im zweiten Reaktionsschritt gibt man die Lösung von 8,0 g (200 mMol) Natriumhydroxyd in 6,5 ml Wasser und 17 ml (20,1 g, 217 mMol) Epichlorhydrin dem Reaktionsgemisch zu, erwärmt auf 75°C, das Dichloräthan verdampft; das System wird schaumig und verfestigt sich dann. Nach dem Abkühlen wird das Reaktionsgemisch gemäß Beispiel 1 gewaschen, getrocknet und das so erhaltene Polymer vermahlen. Man erhält 30,5 g eines Produkts (Proukt C), das einen Cyclodextringehalt von 54,7 g aufweist.

Herstellung von Cyclodextrin-Schaumpolymer mit dem gleichen Wasseraufnahmevermögen nach bekannten Verfahren:

In 50 ml Wasser von 60°C löst man 8,8 g (220 mMol) Natriumhydroxyd, danach 25 g (22 mMol) β-Cyclodextrin und gibt dann 8,5 ml (10,0 g, 1ü8 mMol) Epichlorhydrin zu. In das Reaktionsgemisch mischt man 40 ml Dichloräthan als schaumerzeugendes Mittel ein und führt die weitere Aufarbeitung in der oben angegebenen Weise durch. Es werden 32,0 g eines Produkts (Produkt D) erhalten, das einen Cyclodextringehalt von 52,2 % aufweist.

**Beispiel 3**

In 250 ml Wasser von 60°C löst man 75,0 g (1,88 mMol) Natriumhydroxyd, dann 125 g (0,11 Mol) β-Cyclodextrin und mischt danach 75,0 ml (84,4 g, 0,43 Mol) Tetraäthylenglykol zu. Dem erhaltenen Reaktionsgemisch gibt man zunächst 90 ml (106,2 g, 1,15 Mol) Epichlorhydrin innerhalb von einer Stunde und danach 125 ml Toluol und weitere 52,5 ml (62,ü g, 0,67 Mol) Epichlorhydrin zu. Das Reaktionsgemisch wird drei Stunden intensiv gerührt, wobei die Temperatur bei 60°C gehalten wird. Das erhaltene Produkt besteht aus festen Perlen, die mit Wasser gewaschen, durch Behandlung mit 5 l Aceton dehydratisiert und danach im Trockenschrank bei 120°C getrocknet werden. Es werden 160 g eines Produkts (Produkt E) erhalten, das einen Cyclodextringehalt von 49,0 % aufweist.

In gleicher Weise, aber ohne Tetraäthylenglykol, werden 144 g eines Polymers (Produkt F) hergestellt, dessen Cyclodextringehalt 49,7% beträgt.

**Beispiel 4**

In 10 ml Wasser von 60°C löst man 3,0 g (75 mMol) Natriumhydroxyd, sodann 5,0 g (4,4 mMol) β-Cyclodextrin und gibt einen der Stoffe der Tabelle IV in der dort ancegebenen Menge unter Rühren zu. Um den Polyvinylalkohol zu lösen, muß man mindestens eine weitere halbe Stunde rühren, bei den Zusätzen mit niedrigem Molekulargewicht kann das Reaktionsgemisch schnell homogenisiert werden. Dem Reaktionsgemisch werden dann 3,6 ml (45,9 mMol) Epichlorhydrin innerhalb von einer Stunde zugegeben und danach weitere 2,1 ml (26,7 mMol) Epichlorhydrin zugemischt.

Die Reaktionstemperatur wird stets bei 60°C gehalten. Die Gelbildung erfolgt in 10 bis 90 Minuten, was von der Art und Menge des Zusatzes abhängig ist. Nach dem Abkühlen wird das Reaktionsgemisch mit verdünnter Salzsäure neutralisiert und das so erhaltene Polymer mit Wasser gewaschen und durch Behandlung mit Aceton/Wasser-Gemischen steigender Konzentration dehydratisiert. Das so erhaltene lufttockene Produkt wird im Trockenschrank bei 105°C getrocknet und danach auf die gewünschte einheit ( < 100μ m) vermahlen, wobei die Produkte G bis R erhalten werden.

Zur Prüfung der nach den oben angegebenen Verfahren hergestellten Produkte A bis R wurden die folgenden Vergleichsuntersuchungen durchgeführt.

Aus der folgenden Zusammensetzung:

| | |
|---|---|
| Produkte A bis R | 2,5 Gew.% |
| Mikrokristalline Cellulose (Avicel pH 102) | 94,0 Gew.% |
| Talkum | 1,5 Gew.% |
| Magnesiumstearat | 2,0 Gew.% |

wurden Tabletten unter Anwendung eines Preßdrucks von 0,1 MPa bzw. 100 MPa hergestellt und die Zerfallszeiten und/oder die Wasseraufnahme der Tabletten bestimmt. Die Ergebnisse sind in den folgenden Tabellen zusammengestellt.

**Tabelle I**

Wasseraufnahme von Tabletten
(Tablettenpreßdruck: 0,1 MPA)
ml/g

| | Produkt A | Produkt B |
|---|---|---|
| | | (Beispiel 1) |
| innerhalb von 1 Minute | 1,7 | 0,5 |
| innerhalb von 10 Minuten | 4,9 | 2,0 |
| Endwert | 5,5 | 5,5 |
| Zeit bis zum Erreichen des Endwerts | 16 Minuten | 60 Minuten |

Wasseraufnahme von Tabletten
(Tablettenpreßdruck: 100 MPa)
ml/g

| | Produkt A | Produkt B |
|---|---|---|
| innerhalb von 1 Minute | 0,5 | 0,2 |
| innerhalb von 10 Minuten | 3,7 | 1,0 |
| Endwert | 7,0 | 7,0 |
| Zeit bis zum Erreichen des Endwerts | 28 Minuten | 200 Minuten |

**Tabelle II**

Wasseraufnahme von Tabletten
(Tablettenpreßdruck: 0,1 MPa)
ml/g

| | Produkt C | Produkt D |
|---|---|---|
| | | (Beispiel 2) |
| innerhalb von 1 Minute | 1,9 | 1,5 |
| innerhalb von 10 Minuten | 4,8 | 3,2 |
| Endwert | 5,1 | 5,5 |
| Zeit bis zum Erreichen des Endwerts | 12 Minuten | 33 Minuten |

Wasseraufnahme von Tabletten
(Tablettenpreßdruck: 0,1 MPa)
ml/g

| | Produkt C | Produkt D |
|---|---|---|
| innerhalb von 1 Minute | 0,8 | 0,7 |
| innerhalb von 10 Minuten | 4,3 | 3,0 |
| Endwert | 5,4 | 6,4 |
| Zeit bis zum Erreichen des Endwerts | 15 Minuten | 80 Minuten |

**Tabelle III**

| Wasseraufnahme von Tabletten (Tablettenpreßdruck: 0,1 MPa) ml/g | Produkt E | Produkt F (Beispiel 3) |
|---|---|---|
| innerhalb von 0,5 Minute | 4,3 | 3,4 |
| innerhalb von 1 Minute | 6,0 | 4,4 |
| Endwert | 6,8 | 4,7 |
| Zeit bis zum Erreichen des Endwerts | 1,33 Minuten | 1,15 Minuten |

| Wasseraufnahme von Tabletten (Tablettenpreßdruck: 100 MPa) ml/g | | |
|---|---|---|
| innerhalb von 1 Minute | 1,1 | 1,2 |
| innerhalb von 5 Minuten | 4,0 | 3,4 |
| Endwert | 7,0 | 4,2 |
| Zeit bis zum Erreichen des Endwerts | 12 Minuten | 8 Minuten |

**Tabelle IV**

| Alkoholkomponente (Produkt) | Mol Alkohol-komponente/Mol Epichlorhydrin | Wasseraufnahme von Tabletten Tablettenpreßdruck 0,1 MPa (ml/g) | 100 MPa | Zerfalls-zeit (Minuten) |
|---|---|---|---|---|
| Polyvinylalkohol (Produkt G) | 0,16*) | 4,0 | 3,5 | 4 |
| (Produkt H) | — | 3,7 | 3,5 | 6 |
| n-Propanol (Produkt I) | 0,20 | 4,1 | 4,0 | 2,5 |
| n-Butanol (Produkt J) | 0,20 | 4,6 | 4,8 | 4 |
| n-Octanol (Produkt K) | 0,20 | 4,4 | 5,8 | 4 |
| Äthylenglykol (Produkt L) | 0,22 | 6,6 | 5,9 | 2,5 |
| Propylenglykol (Produkt M) | 0,30 | 4,9 | 5,4 | 2 |
| Butylenglykol (Produkt N) | 0,31 | 6,9 | 8,7 | 2 |
| Triäthylenglykol (Produkt O) | 0,21 | 5,9 | 5,3 | 2 |
| Tetraäthylenglykol (Produkt P) | 0,40 | 9,7 | 10,0 | 2 |
| Glycerin (Produkt R) | 0,15 | 5,0 | 5,2 | 2 |

*) Das Molverhältnis des Polyvinylalkohols ist auf Vinylalkoholeinheiten bezogen.

**0 119 420**

**Patentansprüche** für die Vertragsstaaten, CH, DE, FR, GB, LI

1. In Wasser schnell und stark quellende Cyclodextrin-polymere, aus Cyclodextrin und Epichlorhydrin, die als Zusatzkomponente einen $C_2$-$C_8$-Alkanol, ein Glykol-Derivat oder Glycerin enthalten.

2. - Cyclodextrin-polymere nach Anspruch 1, dadurch gekennzeichnet, daß sie auf 1 Mol Cyclodextrin 2 bis 8 Mol Zusatzkomponente enthalten.

3. Cyclodextrin-polymere nach Anspruch 1 oder 2, dadurch gekennzeichnet, daß sie als $C_2$-$C_8$-Alkanol-Zusatzkomponente n-Propanol, n-Butanol oder n-Octanol enthalten.

4. Cyclodextrin-polymere nach Anspruch 1 oder 2, dadurch gekennzeichnet, daß sie als Glykol-Derivat Äthylenglykol, Propylenglykol, Butylenglykol, Triäthylenglykol oder Tetraäthylenglykol enthalten.

5. Verfahren zur Herstellung von in Wasser schnell und stark quellenden Cyclodextrin-polymeren durch Umsetzung von Cyclodextrin und Epichlorhydrin in wäßrig-alkalischem Medium, dadurch gekennzeichnet, daß man 1 Mol Cyclodextrin mit 10 bis 20 Mol Alkalimetallhydroxyd, 10 bis 20 Mol Epichlorhydrin und 2 bis 8 Mol einer Zusatzkomponente, wie $C_2$- $C_8$-Alkanol, Glykol-Derieines organischen Lösungsmittels, das bei der Reaktionstemperatur verdampft, oder von 8 bis 12 Mol eines hochsiedenden, organischen Lösungsmittels, das eine heterogene Phase sichert, umsetzt.

6. Verfahren nach Anspruch 5, dadurch gekennzeichnet, daß man als $C_2$-$C_8$-Alkanol n-Propanol, n-Butanol oder n-Octanol verwendet.

7. Verfahren nach Anspruch 5, dadurch gekennzeichnet daß man als Glykol-Derivat Äthylenglykol, Propylenglykol, Butylenglykol, Trialkylenglykol oder Tetraäthylenglykol verwendet.

8. Verfahren nach Anspruch 5, 6 oder 7, dadurch gekennzeichnet, daß man als organisches Lösungsmittel, das bei der Reaktionstemperatur verdampft, Äthylenchlorid verwendet.

9. Verfahren nach Anspruch 5, 6 oder 7, dadurch gekennzeichnet, daß man als organisches Lösungsmittel, das eine heterogene Phase sichert, Toluol verwendet.

10. Verwendung der in Wasser schnell und stark quellenden Cyclodextrin-polymeren der Ansprüche 7 bis 4 als Desintegrationsmittel für die Tablettenherstellung.

**Patentansprüche** für den Vertragsstaat AT

1. Verfahren zur Herstellung von in Wasser schnell und stark quellenden Cyclodextrin-polymeren durch Umsetzung von Cyclodextrin und Epichlorhydrin in wäßrig alkalischem Medium, dadurch gekennzeichnet, daß man 1 Mol Cyclodextrin mit 10 bis 20 Mol Alkalimetallhydroxyd, 10 bis 20 Mol Epichlorhydrin und 2 bis 8 Mol einer Zusatzkomponente, wie $C_2$-$C_8$-Alkanol, Glykol-Derivat oder Glycerin, gegebenenfalls in Gegenwart von 10 bis 15 Mol eines organischen Lösungsmittels, das bei der Reaktionstemperatur verdampft, oder von 8 bis 12 Mol eines hochsiedenden, organischen Lösungsmittels, das eine heterogene Phase sichert, umsetzt.

2. Verfahren nach Anspruch 1, dadurch gekennzeichnet, daß man als $C_2$-$C_8$-Alkanol n-Propanol, n-Butanol oder n-Octanol verwendet.

3. Verfahren nach Anspruch 1. dadurch gekennzeichnet, daß man als Glykol-Derivat Äthylenglykol, Propylenglykol, Butylenglykol. Triäthylenglykol oder Tetraäthylenglykol verwendet.

4. Verfahren nach Anspruch 1, 2 oder 3. dadurch gekennzeichnet, daß man als organisches Lösungsmittel, das bei der Reaktionstemperatur verdampft, Äthylenchlorid verwendet.

5. Verfahren nach Anspruch 1, 2 oder 3, dadurch gekennzeichnet, daß man als organisches Lösungsmittel, das eine heterogene Phase sichert, Toluol verwendet.

6. Verwendung der gemäß Ansprüchen 1 bis 5 erhaltenen in Wasser schnell und stark quellenden Cyclodextrin-polymeren als Desintegrationsmittel für die Tablettenherstellung.

**Claims** for the contracting states CH, DE,

FR, GB, LI

1. Cyclodextrin polymers obtained from cyclodextrin and epichlorohydrin, which rapidly swell considerably in water, and which contain as an added component a $C_{2-8}$ alkanol, a glycol derivative or glycerol.

2. Cyclodextrin polymers as claimed in claim 1, characterised in that they contain 2 to 8 mol of added component per 1 mol of cyclodextrin.

3. Cyclodextrin polymers as claimed in claim 1 or 2, characterised in that they contain as the added $C_{2-8}$ alkanol component n-propanol, n-butanol or n-octanol.

4. Cyclodextrin polymers as claimed in claim 1 or 2, characterised in that they contain as glycol derivative ethylene glycol, propylene glycol, butylene glycol, triethylene glycol or tetraethylene glycol.

5. Process for preparing cyclodextrin polymers which rapidly swell considerably in water, by reacting cyclodextrin and epichlorohydrin in an aqueousalkaline medium, characterised in that 1 mol of cyclodextrin is reacted with 10 to 20 mol of alkali metal hydroxide, 10 to 20 mol of epichlorohydrin and 2 to 8 mol of an added component such as $C_{2-8}$ alkanol, glycol derivative or glycerol, optionally in the presence of 10 to 15 mol of an

6

organic solvent which evaporates at the reaction temperature, or in the presence of 8 to 12 mol of a high-boiling organic solvent which ensures a heterogeneous phase.

6. Process as claimed in claim 5, characterised in that n-propanol, n-butanol or n-octanol is used as the $C_{2-8}$ alkanol.

7. Process as claimed in claim 5, characterised in that ethylene glycol, propylene glycol, butylene glycol, triethylene glycol or tetraethylene glycol is used as the glycol derivative.

8. Process as claimed in claim 5, 6 or 7, characterised in that ethylene chloride is used as the organic solvent which evaporates at the reaction temperature.

9. Process as claimed in claim 5, 6 or 7, characterised in that toluene is used as the organic solvent which ensures a heterogeneous phase.

10. Use of the cyclodextrin polymers which rapidly swell considerably in water as claimed in claims 1 to 4 as disintegrants for tablet manufacture.

**Claims** for the contracting state: AT

1. Process for preparing cyclodextrin polymers which rapidly swell considerably in water, by reacting cyclodextrin and epichlorohydrin in an aqueousalkaline medium, characterised in that 1 mol of cyclodextrin is reacted with 10 to 20 mol of alkali metal hydroxide, 10 to 20 mol of epichlorohydrin and 2 to 8 mol of an added component such as $C_{2-8}$ alkanol, glycol derivative or glycerol, optionally in the presence of 10 to 15 mol of an organic solvent which evaporates at the reaction temperature, or in the presence of 8 to 12 mol of a high-boiling organic solvent which ensures a heterogeneous phase.

2. Process as claimed in claim 1, characterised in that n-propanol, n-butanol or n-octanol is used as the $C_{2-8}$ alkanol.

3. Process as claimed in claim 1, characterised in that ethylene glycol, propylene glycol, butylene glycol, triethylene glycol or tetraethylene glycol is used as the glycol derivative.

4. Process as claimed in claim 1, 2 or 3, characterised in that ethylene chloride is used as the organic solvent which evaporates at the reaction temperature.

5. Process as claimed in claim 1, 2 or 3, characterised in that toluene is used as the organic solvent which ensures a heterogeneous phase.

6. Use of the cyclodextrin polymers which rapidly swell considerably in water according to claims 1 to 5 as disintegrants for tablet manufacture.

**Revendications** pour les Etats contractants: CH, DE, FR, GB, LI

1. Polymères de la cyclodextrine gonflant rapidement et fortement dans l'eau obtenus à partir de cyclodextrine et d'épichlorhydrine, qui contiennent comme constituants additionnels un alcanol en $C_2$ à $C_8$, un dérivé du glycol ou du glycérol.

2. Polymères de la cyclodextrine selon la revendication 1, caractérisés en ce qu'ils contiennent pour 1 mole de cyclodextrine, 2 à 8 moles de constituants additionnels.

3. Polymères de la cyclodextrine selon la revendication 1 ou 2, caractérisés en ce qu'ils contiennent comme constituants supplémentaires d'alcanol en $C_2$ à $C_8$, du n-propanol, du n-butanol ou du n-octanol.

4. Polymères de la cyclodextrine selon la revendication 1 ou 2, caractérisés en ce qu'ils contiennent comme dérivé du glycol, de l'éthylèneglycol, du propylèneglycol, du butylèneglycol, du triéthylèneglycol ou du tétraéthylèneglycol.

5. Procédé de préparation de polymères de la cyclodextrine gonflant rapidement et fortement dans l'eau, par réaction de la cyclodextrine et de l'épichlorhydrine dans un milieu aqueux-alcalin, caractérisé en ce qu'on fait réagir 1 mole de cyclodextrine avec 10 à 20 moles d'hydroxyde de métal alcalin, 10 à 20 moles d'épichlorhydrine et 2 à 8 moles d'un constituant additionnel, tel qu'un alcanol en $C_2$ à $C_4$, un dérivé du glycol ou le glycérol, le cas échéant en présence de 10 à 15 moles d'un solvant organique qui s'évapore à la température de la réaction, ou de 8 à 12 moles d'un solvant organique de point d'ébullition élevé, qui assure une phase hétérogène.

6. Procédé selon la revendication 5, caractérisé en ce qu'on utilise comme alcanol en $C_2$ à $C_8$, le n-propanol, le n-butanol ou le n-octanol.

7. Procédé selon la revendication 5, caractérisé en ce qu'on utilise comme dérivé du glycol, l'éthylèneglycol, le propylèneglycol, le butylèneglycol, le triéthylèneglycol ou le tétraéthylèneglycol.

8. Procédé selon la revendication 5, 6 ou 7, caractérisé en ce qu'on utilise comme solvant organique, qui s'évapore à la température de la réaction, du chlorure d'éthylène.

9. Procédé selon la revendication 5, 6 ou 7, caractérisé en ce qu'on utilise comme solvant organique, qui assure une phase hétérogène, le toluène.

10. Utilisation des polymères de la cyclodextrine gonflant rapidement et fortement dans l'eau selon les revendications 1 à 4, comme agent désintégrant pour la fabrication de comprimés.

7

**0 119 420**

**Revendications** pour l'Etat contractant: AT

1. Procédé de préparation de polymères de la cyclodextrine gonflant rapidement et fortement dans l'eau par réaction de la cyclodextrine et de l'épichlorhydrine dans un milieu aqueux-alcalin, caractérisé en ce qu'on fait réagir 1 mole de cyclodextrine avec 01 à 20 moles d'un hydroxyde de métal alcalin, 10 à 20 moles d'épichlorhydrine et 2 à 8 moles d'un constituant additionnel, tel qu'un alcanol en $C_2$ à $C_8$, un dérivé du glycol ou le glycérol, le cas échéant en présence de 10 à 15 moles d'un solvant organique qui s'évapore à la température de la réaction, ou de 8 à 12 moles d'un solvant organique de point d'ébullition élevé, qui garantit une phase hétérogène.

2. Procédé selon la revendication 1, caractérisé en ce qu'on utilise comme alcanol en $C_2$ à $C_8$, le n-propanol, le n-butanol ou le n-octanol.

3. Procédé selon la revendication 1, caractérisé en ce qu'on utilise comme dérivé du glycol, l'éthylèneglycol, le propylèneglycol, le butylèneglycol, le triéthylèneglycol ou le tétraéthylèneglycol.

4. Procédé selon la revendication 1, 2 ou 3, caractérisé en ce qu'on utilise comme solvant organique qui s'évapore à la température de la réaction du chlorure d'éthylène.

5. Procédé selon la revendication 1, 2 ou 3, caractérisé en ce qu'on utilise comme solvant organique qui garantit une phase hétérogène, le toluène.

6. Utilisation des polymères de la cyclodextrine gonflant rapidement et fortement dans l'eau selon les revendications 1 à 5, comme agent désintégrant pour la fabrication de comprimés.

8